# EUROPEAN PATENT APPLICATION

(11) **EP 4 052 702 A1**
(43) Date of publication of application: **07.09.2022**
(21) Application number: 21160814.6
(22) Date of filing: 04.03.2021
(51) Int. Cl.: A61K 31/18, A61K 45/06, A61P 25/08

(54) **CXCL8 INHIBITOR AND PHARMACEUTICAL COMPOSITION THEREOF FOR USE IN THE TREATMENT OF SEIZURES**

(71) Applicant: Dompé farmaceutici S.p.a., 20122 Milano (IT)
(72) Inventor: ALLEGRETTI, Marcello, 67100 L'Aquila (IT); BRANDOLINI, Laura, 67100 L'Aquila (IT); VEZZANI, Annamaria, 20156 Milano (IT); DI SAPIA, Rossella, 20156 Milano (IT); RAVIZZA, Teresa, 20156 Milano (IT); BALOSSO, Silvia Maria, 20156 Milano (IT)
(74) Representative: Mauri, Elisabetta Maria Ester

(57) **Abstract**

The present invention relates to a CXCL8 inhibitor and a pharmaceutical composition thereof, for use in the prevention or treatment of seizures.

## Description

### FIELD OF THE INVENTION

The present invention relates to a CXCL8 inhibitor, such as reparixin, and a pharmaceutical composition thereof for use in the treatment of seizures.

### STATE OF THE ART

A seizure is an abnormal, unregulated electrical discharge that occurs within the brain's cortical gray matter and transiently interrupts normal brain function.

Epilepsy is a neurological disorder affecting about 50 milion of people wordwide characterized by an enduring predisposition to generate unprovoked epileptic seizures and by the associated cognitive, psychological and social consequences (Fisher et al, Epilepsia. 2014, 55: 475-482).

An unprovoked seizure is a seizure occurring in the absence of an acute precipitating factor. Unprovoked seizures include events occurring both in patients with a genetic etiology, without a recognized etiological factor (cryptogenic seizures), or in patients who have had an antecedent central nervous system insult, whose acute phase has been resolved (unprovoked, remote symptomatic seizures).

Symptomatic seizures may also occur in the absence of epilepsy in close temporal relationship with an acute insult to the central nervous system (CNS) (acute symptomatic seizures, ASS) (Beghi et al, Epilepsia 2010, 51(4):671-675). Close temporal association is usually defined as within 1 week of the insult. These seizures represent 40-50% of all cases of seizures and may not recur when the underlying cause has been removed or the acute phase has elapsed.

Symptomatic seizures may be associated to many types of brain insults, , which may be metabolic, toxic, structural, infectious or inflammatory, such as, for example, cerebrovascular disease, brain tumors, alcohol abuse, traumatic head traumas, stroke and infections of the nervous system (Scheffer et al, Epilepsia. 2017, 58: 512-521). The onset of a seizure can be focal (when abnormal brain activity arises in one or more localized brain regions) or generalized when paroxysmal changes begin in a widespread distribution over both hemispheres, or of unknown onset (if available clinical data cannot identify the origin). It is generally assumed that the hyperactivity in neuronal networks during the condition is due to an imbalance wherein excitatory neurotransmission predominates through glutamatergic signaling on inhibitory GABAergic neurotransmission. However, therapeutic anti-seizure strategies targeting these mechanisms have been proven insufficient in a significant proportion of patients (Eyo et al, Glia. 2017, 65(1): 5-18) and it has emerged that the pathophysiological mechanisms underlying the disease are likely to be more complex.

Most seizures last no more than one minute. Occasionally, however, a seizure does not spontaneously terminate and can develop into status epilepticus (SE), which is defined as one seizure that lasts longer than five minutes, or more than one seizure within a five minutes period, without returning to a normal level of consciousness between episodes. Status epilepticus is a life-threatening medical emergency that can cause severe neurological damage, including hippocampal and extra-hippocampal neuron loss.

Early treatment of status epilepticus with benzodiazepines, such as diazepam, is the first line clinical treatment for status epilepticus. However, about a third of patients with status epilepticus may have seizures that are refractory to the first-line medications. In this case, a more aggressive management is required with antiseizure drugs (ASDs), such as phenytoin and valproic acid (VPA), as second-line medications, and anesthetics, such as propofol and pentobarbital, as third line medications.

Status epilepticus may occur in subjects with an established epilepsy or as a consequence of an acute insult to the brain, or for unknown causes. Status epilepticus not associated to an established epilepsy is referred to as de novo status epilepticus and constitutes about 50% of first episodes of status epilepticus.

It has been observed that the occurrence of a de novo status epilecticus in a subject increases the risk for subsequent unprovoked seizure and development to epilepsy compared to less prolonged acute symptomatic seizure (Hesdorffer et al, Ann Neurol 1998; 44:908-9 12).

Epileptogenesis is the process linking an acquired brain injury or other predisposing factors to the subsequent emergence of epilepsy.

Epileptogenesis can be operationally divided in three main phases, although the process should be viewed as a continuum of changes: first, the acute-subacute phase after the occurrence of a precipitating injury or triggering event; second, a 'latent' period during which a previously normal brain network is functionally altered toward decreased seizure threshold, thus having an enhanced probability to generate spontaneous recurrent seizures (SRSs); and third, the establishing of chronic epilepsy (Golberg et al, Nature Reviews 2013, p 337-349).

The changes occurring in the brain during epileptogenesis are complex and dynamic, and may include neurodegeneration, neurogenesis, gliosis, axonal damage or sprouting, dendritic plasticity, blood-brain barrier (BBB) damage, neuroinflammation, recruitment of inflammatory cells into brain tissue, reorganisation of the extracellular matrix, and reorganisation of the molecular architecture of individual neuronal cells (Pitkänen et al, Lancet Neurol 2011, 10: 173-186; Devinsky et al, Nat Rev Dis Primers 2018 ;4: 18024). Substantial research has focused on elucidating the mechanisms of epileptogenesis so as to identify more specific targets for intervention, with the hope of preventing epilepsy before seizures emerge, or improving the disease course. In particular, evidence suggests that the process of acquired epileptogenesis develops beyond the latent period, and includes the development of the disease after its diagnosis, thus extending the window of therapeutic intervention for either preventing the onset of seizures or to interfere with the progression of epilepsy, or both.

The use of available anticonvulsants, which can terminate an ongoing seizure and decrease the occurrence of future seizures in individuals with epilepsy, have been shown to be poorly effective in preventing the process of epileptogenesis. Moreover, these drugs do not affect established seizures in up to 30-40% of epilepsy patients, the so called pharmacoresistant epilepsy population (Devinsky et al, Nat Rev Dis Primers 2018, 4: 18024) A number of potential antiepileptogenic drugs, targeting molecular pathways involved in epileptogenic maladaptive plasticity, such as anti-inflammatory, antioxidant and immunosuppressant drugs, antibodies blocking adhesion of leukocytes to endothelial cells, or drugs restoring the function of blood brain barrier which is compromised in epilepsy etc have been investigated. These treatments are aimed at counteracting epileptogenesis thus preventing or delaying the development of epilepsy, or reducing seizure frequency or duration in the chronic phase.

Temporal lobe epilepsy (TLE) is among the most drug refractory types of epilepsy in adulthood. It is characterized by seizures originating from the limbic system, particularly the hippocampus, parahippocampal regions, and amygdala. TLE is typically considered to be an acquired epilepsy syndrome that is triggered by a precipitating factor, such as head trauma, infection or tumour, or an episode of prolonged, uncontrolled seizure (status epilepticus), although there are known familial forms of TLE.

Animal models of TLE have been a useful tool for clarifying the pathophysiology of epilepsy and for the development of antiepileptic drugs. In particular, the the intra-amygdala model of TLE belongs to a group of animal models that replicate the general progression of events as observed in humans, with 3 distinct phases: (a) an acute-subacute period that includes and follows a limbic status epilepticus, and that lasts for 24 h to 48 h, (b) a latent period devoid of epileptic seizures which lasts for 5-7 days, followed by the onset of epileptic seizures; and (c) a chronic period (1.5-2.5 months post-status epilepticus) with spontaneous recurrent seizures (SRSs) (Mouri et al, Brain Res 2008;1213:140-151; lori et al, Neurobiol Dis 2017; 99: 12-23.; Frigerio et al, Brain. 2018;141:3130-3143).

### SUMMARY OF THE INVENTION

The present inventors have identified a new class of compounds that are able to effectively treat both unprovoked and acute symptomatic seizures and protect the central nervous system from the neurological consequences thereof.

In particular, as will be shown in details in the experimental section, the present inventors have found that in a murine model of TLE, a CXCL-8 inhibitor (reparixin) is effective in reducing the duration of status epilepticus, the incidence of acute symptomatic seizures post-status epilepticus and the acute neuronal cell injury induced therefrom as well as in delaying the onset of epilepsy. Furthermore, reparixin shows an anticonvulsive activity on spontaneous recurrent seizures in established epilepsy.

Accordingly, a first aspect of the invention is a CXCL8-inhibitor for use in the prevention and/or treatment of seizures in an individual.

A further aspect of the invention is a CXCL8-inhibitor for use in the prevention of the onset or progression of epilepsy in an individual.

A further aspect of the present invention relates to a pharmaceutical composition comprising a CXCL8 inhibitor and at least one inert pharmaceutically acceptable excipient, for use in the prevention and/or treatment of seizures or in the prevention of the onset or progression of epilepsy in an individual.

A further aspect of the present invention relates to a method of preventing and/or treating seizures or of preventing the onset or progression of epilepsy in an individual comprising administering to the patient an effective amount of a CXCL8 inhibitor.

A further aspect of the present invention relates to a CXCL8 inhibitor for use in the prevention and/or treatment of seizures or in the prevention of the onset or progression of epilepsy in an individual administered in combination with at least another drug.

A further aspect of the present invention relates to a method of preventing and/or treating seizures or of preventing the onset or progression of epilepsy in an individual, comprising administering to the patient a CXCL8 inhibitor.

### BRIEF DESCRIPTION OF THE FIGURES

Figure 1 (A - C) shows cumulative data from mice exposed to SE and treated with either vehicle or reparixin;
Figure 2 (A - C) shows the the incidence of acute symptomatic seizures during 48 h post-SE in all animals exposed to SE (A), the onset time of epilepsy (B) and the total number of spontaneous seizures (C) in the mice treated with either vehicle or reparixin;
Figure 3 (A - B) shows the total number of spontaneous recurrent seizures (SRS) (A) and their average duration (B) during 2 weeks of EEG recording at 2.5 months post-SE in mice.Figure 4 (A - B) shows representative sections stained for detection of acetylated alpha -tubulin in the CA1 pyramidal cell layer (A) and signal quantification in CA1, CA3 pyramidal cell layers and hilus (B);
Figure 5 shows the number of Fluoro-Jade-positive degenerating neurons in CA1, CA3, CA4 pyramidal cell layers and hilus in mice exposed to SE and treated with reparixin or vehicle;
Figure 6 (A - C) shows the number of seizures (A), their average duration (B) in chronic epileptic mice treated with reparixin or vehicle and the waterfall plot (C) on reparixin effect in each epileptic mouse during treatment and wash out.

### DEFINITIONS

According to the present invention, the term "prevention" refers to administration to an individual to obtain partial or complete prevention of a disorder or pathological event, before this is established or occurs.

According to one embodiment of the invention, prevention is a complete prevention, wherein the disorder or pathological event is completely blocked.

According to an alternative embodiment of the invention, prevention is a partial prevention, wherein the development of the disorder or pathological event is delayed and/or its severity reduced.

According to the present invention, the term "treatment" refers to complete reversal or reduction of severity or progression of a disorder or pathological event, after this is established or occurs.

According to the present invention, the term "individual" refers to a human or an animal being, preferably to a human being.

### DETAILED DESCRIPTION OF THE INVENTION

A first aspect of the present invention relates to a CXCL8-inhibitor, a pharmaceutically acceptable salt therof or a prodrug thereof, for use in the prevention or treatment of seizures in an individual.

According to one preferred embodiment, said seizures are acute symptomatic seizures (ASS).

In a form of realization of the invention according to this embodiment, the CXCL8-inhibitor is for use in the prevention of acute symptomatic seizures in an individual that is or has been subjected to an acute insult to the central nervous system. In such instance, the CXCL8 inhibitor is administered as soon as possible during or after the insult.

In an alternative form of realization of the invention according to this embodiment, the CXCL8-inhibitor is for use in the treatment of acute symptomatic seizures in an individual. In such instance, the CXL8 inhibitor is administered during or as soon as possible after occurrence of said acute symptomatic seizures.

According to another preferred embodiment, said seizures are associated to an established epilepsy (Established Epilepsy Seizures EES).

In one form of realization of the invention according to this embodiment, the CXCL8-inhibitor is for use in the prevention of seizures associated to an established epilepsy. Preferably, according to this form of realization, said CXCL8 inhibitor is administered chronically to an individual with established epilepsy.

In an alternative form of realization of the invention according to this embodiment, the CXCL8-inhibitor is for use in the treatment of seizures associated to an established epilepsy (Established Epilepsy Seizures EES). In such instance, the CXL8 inhibitor is administered during or after occurrence of said seizures.

According to another preferred embodiment, said seizures are (Status Epilepticus Seizures, SES).

According to this embodiment said status epilepticus may be associated or not associated (de novo status epilepticus) to the presence of an established epilepsy.

In a form of realization of the invention according to this embodiment, the CXCL8-inhibitor is for use in the prevention of de novo status epilepticus seizures in an individual that is or has been subjected to an acute insult to the central nervous system. In such instance, the CXL8 inhibitor is administered during or as soon as possibile after the insult.

In another form of realization of the invention according to this embodiment, the CXCL8-inhibitor is for use in the prevention of status epilepticus seizures in an individual having an established epilepsy. In such instance, the CXL8 inhibitor is administered chronically to individuals having an established epilepsy and being at risk of developing status epilepticus.

In an alternative form of realization of the invention according to this embodiment, the CXCL8-inhibitor is for use in the treatment of status epilepticus seizures, associated or not associated with epilepsy, in an individual. In such instance, the CXL8 inhibitor is administered during occurrence of said status epilepticus seizures.

The prevention or treatment of acute symptomatic seizures and/or status epilepticus seizures according to the present invention is useful in preventing the onset of epilepsy or the progression of an established epilepsy in an individual.

Accordingly, a further aspect of the present invention relates to a CXCL8-inhibitor for use in the prevention of the onset of epilepsy or the progression of an established epilepsy in an individual.

Preferably, the CXCL8 inhbitor for use according to the invention is preferably administered in accordance with the treatment guidelines for the specific condition of the individual treated, that may require the administration in combination with other drugs. According to one embodiment, the CXCL8 inhibitor for use according to the invention is administered in combination with one or more other drugs, preferably selected from benzodiazepines and antiseizure drugs (ASDs). Said other drugs may be administered simultaneously, sequentially or separately.

According to a preferred embodiment, said benzodiazepines administered in combination with the CXCL8 according to the invention are selected from diazepam and lorazepam and said antiseizure drugs are selected from phenytoin and valproic acid (VPA).

The term CXCL8-inhibitor according to the present application refers to any compound able to inhibit the biological activity of CXCL8.

According to a preferred embodiment, the CXCL8 inhibitor according to the present invention is a CXCL8 receptor(s) inhibitor. Preferably said CXCL8 receptor(s) inhibitor is a CXCR1- or a CXCR1/2-inhibitor, which inhibits the activity of CXCL8 mediated by CXCR1 receptor or mediated by both the CXCR1 and CXCR2 receptors.

Preferably, according to this embodiment, said CXCL8 receptor(s) inhibitor inhibits binding of CXCL8 to the CXCR1 receptor or to both the CXCR1 and CXCR2 receptors. or blocks the intracellular signaling activated by the binding of CXCL8 to the CXCR1 receptor or both the CXCR1 and CXCR2 receptors.

Preferably, the CXCL8 receptor(s) inhibitor is an antagonists of the CXCR1 receptor or of both CXCR1 and CXCR2 receptors. More preferably, said CXCL8 receptor(s) inhibitor is an allosteric inhibitor or an orthosteric antagonist of CXCR1 receptor or of both CXCR1 and CXCR2 receptors.

Alternatively, preferably the CXCL8 receptor(s) inhibitor binds to CXCL-8 thus preventing its binding to its receptors.

It is preferred that said CXCL8 receptor inhibitor(s) is able to inhibit in an in vitro assay at least 60%, preferably at least 70%, more preferably at least 80%, even more preferably at least 90% of PMNs chemotaxis induced by 1nM CXCL8 at a concentration equal or below 500 nM, preferably below 100 nM.

More preferably, the CXCL8 receptor(s) inhibitor according to the invention has an IC₅₀ value towards CXCR1 receptor in the low nanomolar range, preferably lower than 10 nanomolar, more preferably in the range 0.02-5 nanomolar.

According to a preferred embodiment, also in combination with any one of the preceding embodiments, said CXCL8 inhibitor is selected from small molecules, peptides and antibodies, more preferably it is a small molecule.

The term small molecule refers to an organic compound having a molecular weight of 900 Daltons or lower.

CXCL8 inhibitors, in particular CXCL8 receptor(s) inhibitors, according to the above definition, are well known in the art.

To date, several CXCL8 inhibitors, such as small molecules, peptides and antibodies, have been disclosed, many of which are currently undergoing clinical trials or are used in therapy. (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78).

Preferably, said CXCL8 receptor(s) inhibitors for use according to the invention is selected from a list consisting of:
- the anti-CXCL-8 antibodies ABCream, BMS-986253 and ABX-IL-8;
- RP-72, PAC-G-31-P, SCH-N, Navarixin, having formula 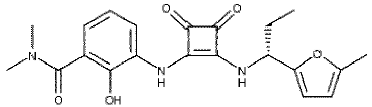 SX-517, having formula 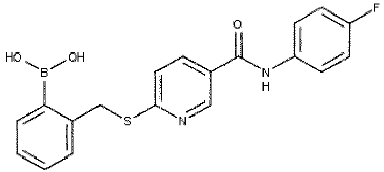 SX-576, having formula 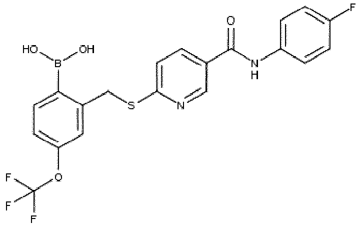 compounds having formula 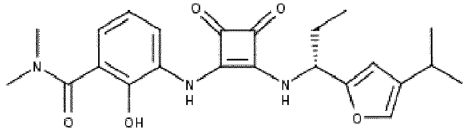 or 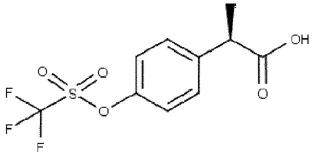 or 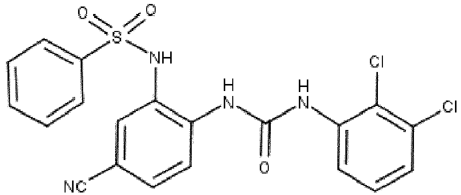 and
   5-[3-(2-Fluorophenyl)ureido]-1-(2-hydroxypropyl)-1H-pyrazole-4-carboxylic acid ethyl ester
   5-[3-(3-Fluorophenyl)ureido]-1-(2-hydroxypropyl)-1H-pyrazole-4-carboxylic acid ethyl ester
   3-[2-[1(R)-(4-Bromofuran-2-yl)propylamino]-3,4-dioxo-1-cyclobutenylamino]-2-hydroxy-N,N-dimethylbenzamide
   3-[2-[1(R)-(4-Chlorofuran-2-yl)propylamino]-3,4-dioxo-1-cyclobutenylamino]-2-hydroxy-N,N-dimethylbenzamide
   Trifluoromethanesulfonic acid 4-[1(R)-(N-isopropylcarbamoyl)ethyl]phenyl ester 2-Hydroxy-3-[4-[1 (R)-(4-isopropylfuran-2-yl)propylamino]-1-oxo-1,2,5-thiadiazol-3-ylamino]-N,N-dimethylbenzamide
   3-(2-Chlorophenylamino)-7-nitro-4H-1,2,4-benzothiadiazin-5-ol 1,1-dioxide 1-[3-[4-[3-(4-Fluorophenyl)isoxazol-5-yl]phenoxy]propyl]-4-methylpiperazine N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) described hereinbelow.

CXCL8 inhibitors particularly preferred in the present invention are CXCL8 receptor(s) inhibitors of following formula (I): 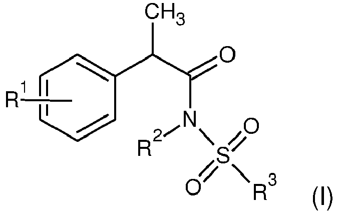 wherein
R¹ is selected from a linear or branched C₁-C₆ alkyl, benzoyl, phenoxy, and trifluoromethanesulfonyloxy;
R² is selected from hydrogen atom and linear or branched C₁-C₃ alkyl; and
R³ is a linear or branched C₁-C₆ alkyl or trifluoromethyl,
or a pharmaceutically acceptable salt thereof,
as those described in WO2000/024710A1 and WO2005/090295A2.

Preferably, R¹ is selected from benzoyl, isobutyl, and trifluoromethanesulfonyloxy. More in particular, R¹ is preferably linked to the phenyl ring in 3- or 4-position. According to the most preferred embodiment, R¹ is 3-benzoyl, 4-isobutyl or 4-trifluoromethanesulfonyloxy. Preferably, R² is selected from hydrogen atom or methyl.

Preferably, R³ is selected from linear or branched C₁-C₆ alkyl, more preferably from linear of branched C₁-C₃ alkyl. According to the most preferred embodiment, R³ is methyl. Peferably, the chiral carbon of the compounds of formula (I) is in the RS or R configuration, more preferably it is in the R configuration.

Particularly preferred among said CXCL8 inhibitors of formula (I) are:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof (also known as reparixin), and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, in particular the sodium salt thereof (also known as ladarixin or DF2156A).

Also, other particularly preferred CXCL8 inhibitors according to the present invention are the CXCL8 receptor(s) inhibitors of the following formula (II), described in patent application WO2010/031835A2: 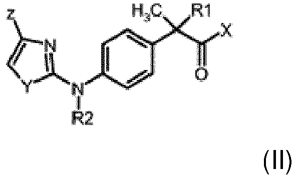 and pharmaceutically acceptable salts thereof, wherein:
R1 is hydrogen;
X is OH;
R2 is hydrogen or linear C1-C4 alkyl,
Y is a heteroatom selected from S, O and N,
Z is selected from linear or branched C1-C4 alkyl, linear or branched C1-C4 alkoxy, halo C1-C3 alkyl and halo C1-C3 alkoxy.

Peferably, the chiral carbon of the compounds of formula (II) is in the RS or S configuration, more preferably in the S configuration.

Particularly preferred among said CXCL8 inhibitors of formula (II) is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

The most preferred CXCL8 inhibitors according to the present invention is R-(-)-2-(4-isobutylphenyl)propionyl methansulfonamide (also known as reparixin) and pharmaceutically acceptable salts thereof, preferably the lysine in situ salt thereof.

The CXCL8 inhibitor compounds of the present invention may form stable pharmaceutically acceptable salts with a pharmaceutically acceptable organic or inorganic acid or base, and in such cases administration of a compound as a salt may be appropriate.

Examples of acid addition salts include acetate, adipate, ascorbate, benzoate, benzenesulfonate, bicarbonate, bisulfate, butyrate, camphorate, camphorsulfonate, choline, citrate, cyclohexyl sulfamate, diethylenediamine, ethanesulfonate, fumarate, glutamate, glycolate, hemisulfate, 2-hydroxyethylsulfonate, heptanoate, hexanoate, hydrochloride, hydrobromide, hydroiodide, hydroxymaleate, lactate, malate, maleate, methanesulfonate, meglumine, 2-naphthalenesulfonate, nitrate, oxalate, pamoate, persulfate, phenylacetate, phosphate, diphosphate, picrate, pivalate, propionate, quinate, salicylate, stearate, succinate, sulfamate, sulfanilate, sulfate, tartrate, tosylate (p-toluenesulfonate), trifluoroacetate, and undecanoate.

Examples of base addition salts include ammonium salts; alkali metal salts such as sodium, lithium and potassium salts; alkaline earth metal salts such as aluminum, calcium and magnesium salts; salts with organic bases such as dicyclohexylamine salts and N-methyl-D-glucamine; and salts with amino acids such as arginine, lysine, ornithine, and so forth. Also, basic nitrogen-containing groups may be quaternized with such agents as: lower alkyl halides, such as methyl, ethyl, propyl, and butyl halides; dialkyl sulfates such as dimethyl, diethyl, dibutyl; diamyl sulfates; long chain halides such as decyl, lauryl, myristyl and stearyl halides; arylalkyl halides such as benzyl bromide and others. Nontoxic physiologically-acceptable salts are preferred, although other salts may be useful, such as in isolating or purifying the product.

The salts may be formed by conventional means, such as by reacting the free form of the product with one or more equivalents of the appropriate acid or base in a solvent or medium in which the salt is insoluble, such as for example water or ethanol, which is removed under vacuum or by freeze drying.

The present invention also includes the prodrugs, stereoisomers, isotope-labelled, for example deuterated, derivatives and enantiomers of the CXCL8 inhibitor compounds described above.

As used herein the term "prodrug" refers to an agent, which is converted into the parent drug *in vivo* by some physiological process (e.g., a prodrug on being brought to the physiological pH is converted to the desired drug form). Prodrugs are often useful because, in some situations, they may be easier to administer than the parent drug. They may, for instance, be bioavailable by oral administration whereas the parent drug is not. The prodrug may also have improved solubility in pharmacological compositions over the parent drug. An example, without limitation, of a prodrug would be a compound of the present invention wherein it is administered as an ester (the "prodrug") to facilitate the transfer across a cell membrane, where water solubility is detrimental, but then it is metabolically hydrolysed once inside the cell where water solubility is beneficial. Prodrugs have many useful properties. For example, a prodrug may be more watersoluble than the ultimate drug, thereby facilitating intravenous administration of the drug. A prodrug may also have a higher level of oral bioavailability than the ultimate drug. After administration, the prodrug is enzymatically or chemically cleaved to deliver the ultimate drug in the blood or tissue.

Ester prodrugs of the CXCL8 inhibitor compounds disclosed herein are specifically contemplated. While not intending to be limiting, an ester may be an alkyl ester, an aryl ester, or a heteroaryl ester. The term alkyl has the meaning generally understood by those skilled in the art and refers to linear, branched, or cyclic alkyl moieties. C₁₋₆ alkyl esters are particularly useful, where alkyl part of the ester has from 1 to 6 carbon atoms and includes, but is not limited to, methyl, ethyl, propyl, isopropyl, n-butyl, sec-butyl, iso-butyl, t-butyl, pentyl isomers, hexyl isomers, cyclopropyl, cyclobutyl, cyclopentyl, cyclohexyl, and combinations thereof.

Certain CXCL8 inhibitors may exist in tautomeric forms, and this invention includes all such tautomeric forms of those compounds unless otherwise specified.

Unless otherwise stated, structures depicted herein are also meant to include all stereochemical forms of the structure; i.e., the R and S configurations for each asymmetric centre. Thus, single stereochemical isomers as well as enantiomeric and diastereomeric mixtures of the present compounds are within the scope of the invention. Thus, this invention encompasses each diastereomer or enantiomer substantially free of other isomers (>90%, and preferably >95%, free from other stereoisomers on a molar basis) as well as a mixture of such isomers in any ratio.

Particular optical isomers can be obtained by resolution of the racemic mixtures according to conventional processes, e.g., by formation of diastereomeric salts, by treatment with an optically active acid or base or enzymatically. Examples of appropriate acids are tartaric, diacetyltartaric, dibenzoyltartaric, ditoluoyltartaric, and camphorsulfonic acid and then separation of the mixture of diastereomers by crystallization followed by liberation of the optically active bases from these salts. A different process for separation of optical isomers involves the use of a chiral chromatography column optimally chosen to maximize the separation of the enantiomers. Still another method involves synthesis of covalent diastereomers by reacting compounds of the invention with an optically pure acid in an activated form or an optically pure isocyanate. The synthesized diastereomers can be separated by conventional means such as chromatography, distillation, crystallization or sublimation, and then hydrolysed to deliver the enantiomerically pure compound. Optically active compounds of the invention can be obtained by using active starting materials. These isomers may be in the form of a free acid, a free base, an ester or a salt.

The CXCL8 inhibitors of the present invention may be in amorphous or crystalline form, including any polymorphic form.

Typically, the CXCL8 inhibitor for use according to the first and further aspects of the invention is administered in the form of a pharmaceutical composition.

Accordingly, a further aspect of the present invention relates to a pharmaceutical composition comprising a CXCL8 inhibitor as previously defined and at least one inert pharmaceutically acceptable excipient, for use in the prevention or treatment of seizures or in the prevention of the onset or progression of epilepsy in an individual, as described above.

Preferably, the pharmaceutical composition of the present invention is prepared in suitable dosage forms comprising an effective amount of a CXCL8 inhibitor, a pharmaceutically acceptable salt therof, or a prodrug thereof, and at least one inert pharmaceutically acceptable excipient.

The administration of the pharmaceutical composition of the present invention to a patient is in accordance with known methods and may comprise from one to several oral administrations per day (for example, two times a day (BID) or four times a day (QID)), parenteral administration (including intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques), topical, buccal and suppository administration, or by sustained release systems among other routes of administration.

In the present description and in the following claims, the wording "effective amount" means an amount of a compound or composition which is sufficient to significantly to achieve the desired clinical response.

The effective amount of an active ingredient for use according to the invention will vary with the particular condition associated to the seizures being prevented or treated, the severity of the condition, the duration of the treatment, the nature of concurrent therapy, the particular active ingredient(s) being employed, the particular pharmaceutically-acceptable excipient(s)/carrier(s) utilized, and like factors that are within the knowledge and expertise of the attending physician. Also specific dosage and treatment regimens for any particular patient will depend upon a variety of factors, including for example the activity and half life of the specific compound employed, the age, body weight, general health status, sex, diet, severity and course of the disease.

As described herein, the pharmaceutical composition of the present invention comprises a CXCL8 inhibitor together with a pharmaceutically acceptable excipient, which, as used herein, includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, osmotic regulators, emulsifiers, sweeteners, colorants, flavourings and the like.

The amount of a CXCL8 inhibitor or the pharmaceutically acceptable salt thereof in the pharmaceutical composition of the present invention can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

For example, whent the CXCL8 inhibitor is reparixin the amount of said CXCL8 inhibitor or the pharmaceutically acceptable salt thereof in the pharmaceutical composition of the present invention will be such as to ensure a dosage per single administration of from 400 to 1400 mg, preferably from to 800-1200 mg, even more preferably 1200 mg, to be administered three times a day.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the disease, and the patient's disposition to the disease and the judgment of the treating physician.

The dosage forms of the pharmaceutical composition of the present invention can be prepared by techniques that are familiar to a pharmaceutical chemist, and comprise mixing, granulation, compression, dissolution, sterilization and the like.

In a further aspect, the present invention relates to a method of preventing or treating seizures or of preventing the onset or progression of epilepsy in an individual, as described above, comprising administering to the patient an effective amount of a CXCL8 inhibitor as above defined.

Preferably, in the method according to the invention, said CXCL8 inhibitor is administered in form of a pharmaceutical composition, as above defined.

The invention will be further described in the following examples, which do not limit the scope of the invention defined in the claims.

### EXPERIMENTAL SECTION

### MATERIALS

Phosphate Buffered SalineNaCl Cod. 479687(Sigma Aldrich srl)

Sodium phosphate dibasic Cod. 71640 (Sigma Aldrich srl)

Sodium phosphate monobasic Cod. 60218 (Sigma Aldrich srl)

Kainic Acid Cod. K0250-10m (Sigma Aldrich srl)

Protease inhibitor cocktail Cod. P8340 (Sigma Aldrich srl)

Stop Solution - 1 M Sulfuric acid, Cod. 35276 (Honeywell Fluka)

Sucrose Cod. 84100 (Sigma Aldrich srl)

Paraformaldehyde Cod. 1040051000 (VWR International S.r.l)

Triton X-100 Cod. T8787(Sigma Aldrich srl)

Absolute ethanol Cod. 308662 (CARLO ERBA Reagents)

Xylene Cod. 33817-6X (Sigma Aldrich srl)

Albumin Bovine Serum Cod. A7030 (Sigma Aldrich srl)

VECTASTAIN ABC-PEROXIDASE KIT Standard (Reagent A and B) Cod. PK4000 (VINCI-BIOCHEM srl)

Fluoro Jade B Cod. #1FJB (HISTO-CHEM INC.)

Acetylated alpha-Tubulin Cod. #24610 (Abcam)

Alexa 488 anti-rabbit Cod. a11034 (Life Technologies Italia, Invitrogen)

FLUORSAVE REAGENT - CALBIOCHEM Cod. 345789 (VWR International S.r.l)

KPL SureBlue^{™}, TMB Microwell Peroxidase Substrate (1-Component), Cod. 5120 0075 (Sera Care).

### DATA ANALYSIS

All efforts were made to minimize the number of animals used and their suffering according to the principles of the three Rs (Replacement, Reduction and Refinement; https://www.nc3rs.org.uk/the-3rs). A simple random allocation was applied to assign a subject to a particular experimental group. Statistical analysis was performed by GraphPad Prism 8 (GraphPad Software, USA) for Windows using absolute values. Data are presented as mean ± standard error of the mean (SEM) (n=number of individual samples). Variation of acetylated alpha-tubulin and number of seizures in chronic epileptic mice was analysed by one-way ANOVA with Tukey's post-hoc test. Difference in the duration of SE between control and treated group was analysed by Mann Whitney test for two independent groups. The temporal distribution of spikes was analyzed by two-way ANOVA followed by Bonferroni's multiple comparisons test. The comparison of proportions was done by Fisher test. The onset of epilepsy was analysed by Log-rank test. Differences between groups were reported as statistically significant for values of p<0.05.

### Example 1

### a) Preparation of animals for status epilepticus (SE) induction and electroencephalogram (EEG) recording

A murine model of acquired epilepsy triggered by de novo status epilepticus was used in the experiments (lori et al, Neurobiol Dis 2017, 99 12-23).

In details, eight week-old C57BL6/N male mice (-25 g; Charles River, calco, Italy) were used. Mice were maintained in SPF facilities and housed at a constant room temperature (23°c) and relative humidity (60 ± 5%) with free access to food and water and a fixed 12 h light/dark cycle. All experimental procedures were conducted in conformity with institutional guidelines that are in compliance with national (D.L. n.26, G.U. march 4, 2014) and international laws and policies (EEC council directive 86/609, OJ L 358, 1, December 12, 1987, guide for the care and use of laboratory animals, U.S. national research council, 1996), and were reviewed and approved by the intramural ethical committee.

Mice were surgically implanted under general gas anaesthesia (1-3% isoflurane in O2) and stereotaxic guidance. A 23-gauge guide cannula was unilaterally positioned on top of the dura mater for the intra-amygdala injection of kainic acid (KA) (from bregma, mm: nose bar 0; anteroposterior, -0.9; lateral, -3.1) in accordance with lori et al. (2017).

For seizure recording, a nichrome-insulated bipolar depth electrode (60 µm OD) was implanted in the dorsal hippocampus (from bregma, mm: nose bar 0; anteroposterior - 1.8, lateral 1.4 and 1.8 below dura mater) contralateral to the injected amygdala and a cortical electrode was placed onto the somatosensory cortex in the ipsilateral hemisphere. Finally, two screw electrodes were positioned over the nasal sinus and the cerebellum, and used as ground and reference electrodes, respectively. Electrodes were connected to a multipin socket and secured to the skull by acrylic dental cement.

### b) Implantation of minipumps

Minipumps (Alzet pump 1007D, 100 µl/minipump or Alzet pump 2002, 200 µl/minipump; Charles River, Calco, Italy) filled with reparixin dissolved in a vehicle consisting in sterile saline (NaCl 0.9%) solution (vehicle) (reparixin group) or vehicle (sham control group and vehicle group) were implanted subcutaneously in the back of the mice. Briefly, a small incision was made in the skin between the scapulae and a pocket was formed by spreading apart the connective tissues. After minipump positioning, the skin was carefully sutured to avoid any discomfort to the animal.

One week after surgery, mice were connected to the EEG set up (TWIN EEG recording system connected with a comet AS-40 32/8 amplifier:sampling rate 400 hz, high-pass filter 0.3 hz, low-pass filter 70 hz, sensitivity 2000 mv/cm; Grass-telefactor, West Warwick, USA) in order to record an EEG baseline before the induction of status epilepticus (SE).

### Example 2

### Effect of reparixin on onset and development of epilepsy

A cohort of 29 mice were divided in three experimental groups:
sham group (n=8), vehicle group (n=12) and reparixin group (n=9).

Reparixin, at the dose of 7.5 mg/h/kg (reparixin group), or vehicle (sham and vehicle group) were administered by continuous infusion using osmotic minipumps as described in example 1b).

After 24 hours from infusion, status epilepticus was induced in the vehicle group and reparixin group by amygdal injection of kaininc acid.

In details, kainic acid (KA, 0.3 µg in 0.2 µl; Sigma-Aldrich, S. Louis, USA) was dissolved in 0.1 mM phosphate-buffered saline (PBS, pH 7.4) and injected into the right basolateral amygdala in freely moving mice using a needle protruding 4.1 mm below the implanted cannula in order to evoke SE. SE was defined by the appearance of continuous spike activity with a frequency >1 Hz intermixed with high amplitude and frequency discharges lasting for at least 5 s, with a frequency of >8 Hz. Spikes were defined as sharp waves with amplitude at least 2.5-fold higher than the standard deviation of baseline and duration <100 ms, or as a spike-and-wave with duration <200 ms. SE developed after approximately 10 min from KA injection, as previously described in lori et al. 40 min from SE onset, mice were injected with diazepam (10 mg/kg, intraperitoneally, i.p.) to improve their survival by suppressing motor seizures, although EEG SE was not interrupted. After SE induction mice were recorded continuously (24/7) for 24 h. The end of SE was defined by the occurrence of interspike intervals longer than 1 s. Digitized EEG data were processed using the twin record and review software. SE duration and spiking activity were quantified using clampfit 9.0 program (Axon Instruments).

The mice of the sham group were injected intra amygdala with phosphate buffered saline at the same timepoint.

Spontaneous seizures are EEG paroxysmal events lasting 30-60 sec on average and simultaneously occurring in hippocampus and fronto-parietal cortex, bilaterally.

Mice were EEG recorded continuously (24 h/ 7 days) for 14 days, starting from the day of SE induction (this period includes both epileptogenesis and early disease development). Since acute symptomatic seizures may occur during 48 h after SE, epilepsy onset was defined by the occurrence of spontaneous seizures from 48 h after the end of SE. After 14 days after the induction of SE, mice were temporarily disconnected from the EEG set up, and left in their home cage until they were again monitored (24/7) at 2.5 months post-SE for 2 weeks, corresponding to the chronic epilepsy phase. During the recording period, the total number of spontaneous EEG seizures and the average seizure duration were measured.

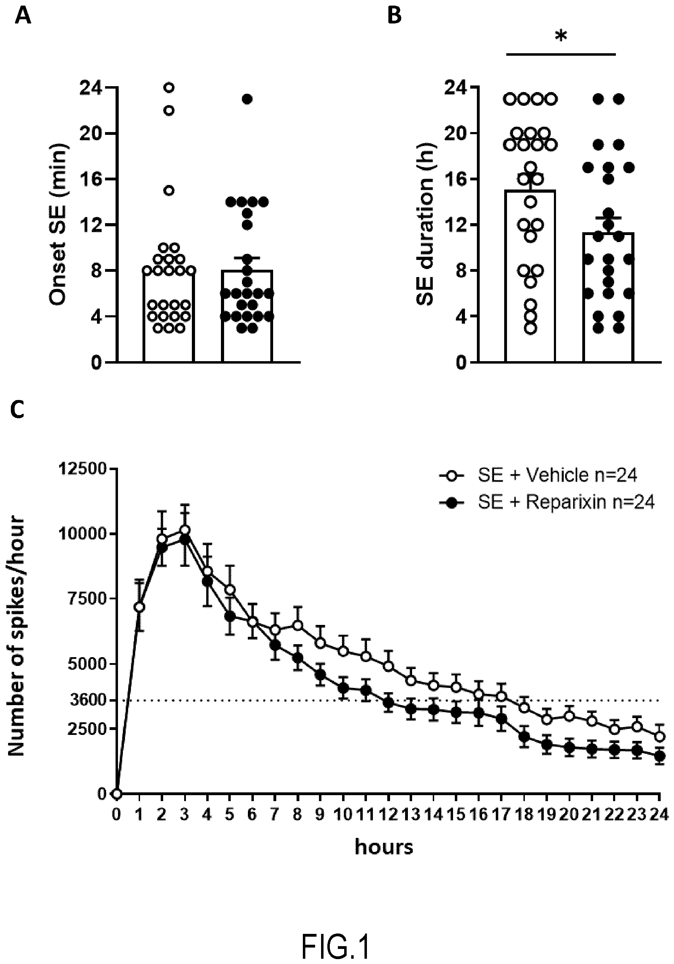

Figure 1 (A-C) reports cumulative data from all mice exposed to SE and treated with either vehicle or reparixin. Figure 1 shows the time to onset of status epilepticus SE (A) and its duration (B) in mice treated with reparixin or vehicle. Figure 1C depicts the temporal spike distribution during SE in both groups. Reparixin administration did not modify the onset time of SE but significantly shortened SE duration when compared to the vehicle group (n=24/group).

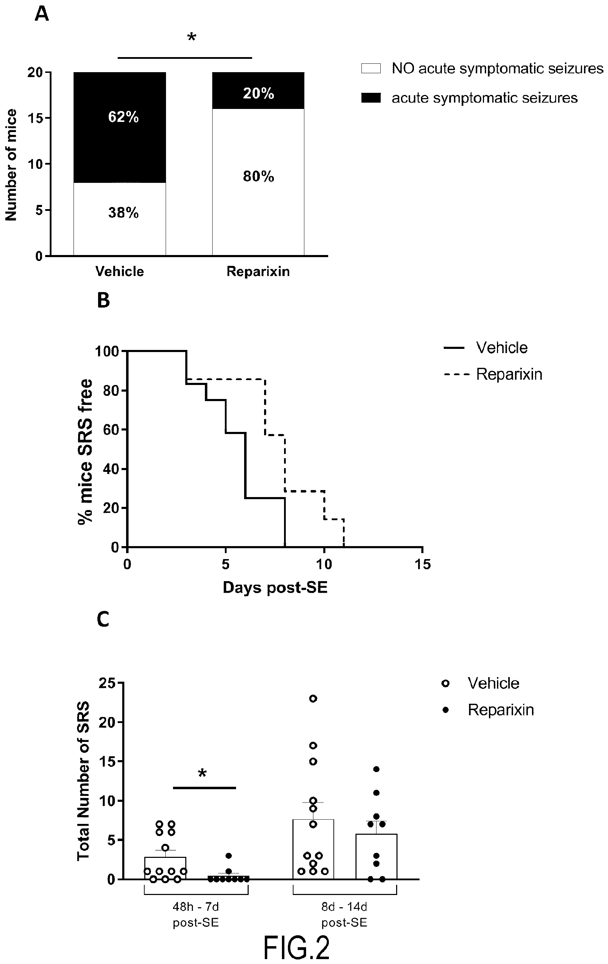

Figure 2A shows the the incidence of acute symptomatic seizures during 48 h post-SE in all animals exposed to SE. Figures 2B and C report, respectively, the onset time of epilepsy (B) and the total number of spontaneous seizures (C) in the mice of the longitudinal study (i.e., mice treated with either vehicle or reparixin for 7 days post-SE;
Example 2): Reparixin treated mice experienced significantly less acute symptomatic seizures (20% of mice; p<0.05 by Fisher's test) as compared to vehicle treated mice (62% of mice).
Figure 2B depicts the Kaplan-Meyer survival curve reporting the percent of mice developing spontaneous recurrent seizures (SRS) as a function of time. Reparixin-treated mice displayed a significant delay in the onset of spontaneous seizures (p<0.05, Log-rank test) vs vehicle-treated mice.
Figure 2C shows the number of spontaneous recurrent seizures (SRS) during drug treatment (48 h - 7 d post-SE) and during drug wash-out (8-14 d post-SE). The reparixin group showed a significant reduction of seizures during treatment vs the vehicle group (p<0.05, Mann-Whitney test) whereas during the wash-out period seizures were similar to the vehicle group.

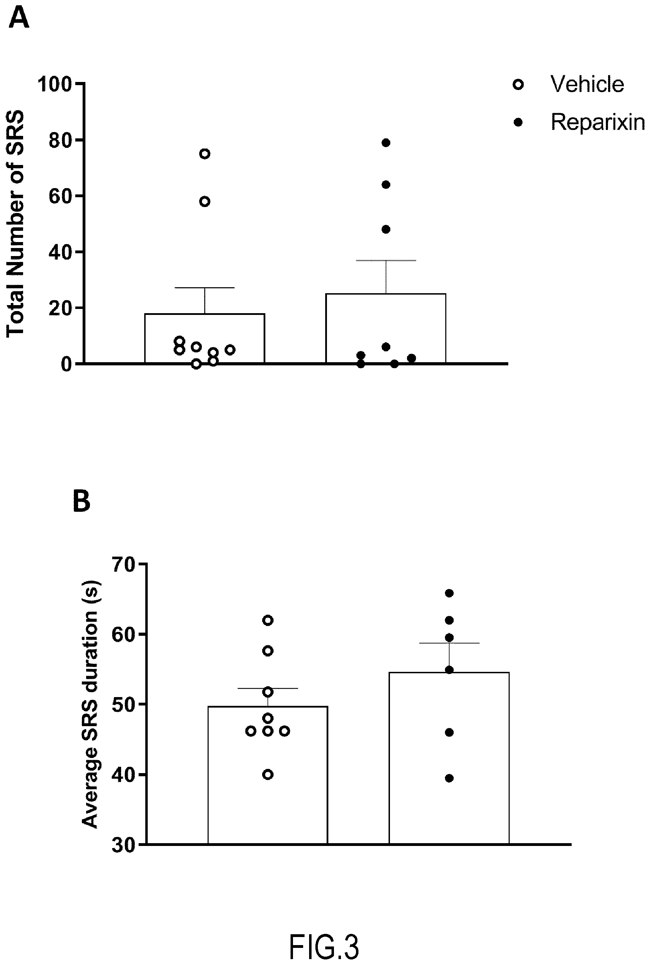

Figure 3 shows the total number of spontaneous recurrent seizures (SRS) (A) and their average duration (B) during 2 weeks of EEG recording at 2.5 months post-SE in the same mice as above: the data show that after drug discontinuation the number of seizures was similar to vehicle-treated mice. During epilepsy development 3 out of 12 mice in the vehicle group died because of severe seizures while 1 out of 9 mice died in the reparixin group.

The obtained results demonstrate that reparixin is effective in reducing SE duration. Furthermore, reparixin shows an anticonvulsive activity with reduction of acute symptomatic seizures developing post-SE and a delay in the development of spontaneous seizures. Finally, reparixin by reducing mortality in mice shows a protective action against neuronal damage associated with convulsions.

### Example 3

### Effect of reparixin on acute neuronal injury induced by SE

A cohort of animals was prepared in accordance to Example 1 to study the effects of reparixin on the acetylated alpha-tubulin, a pharmacodynamic marker of drug's neuroprotective effect, and on degenerating neurons, as identified by Fluoro-Jade staining.

In details, the mice were divided in two experimental groups:
vehicle group (n=6) and reparixin group (n=7). Sham mice (implanted with electrodes but not exposed to SE) were used as controls (n=6).

The animals of the different groups were treated as described in Example 2.

All mice were transcardially perfused 72 h post-SE with ice-cold phosphate buffered saline (PBS, pH 7.4), followed by 4% paraformaldehyde (PFA) in PBS. Brains were post-fixed for 90 min in 4% PFA in PBS at 4°C, then transferred to 20% sucrose in PBS overnight at 4°C and then frozen in n-pentane for 3 min at -43°C and stored at -80°C until assay. Serial coronal sections (40 µm) were cut on a cryostat throughout the extension of the hippocampus (from bregma -1.22 to -2.54 mm).

Sections were processed to measure both degenerating neurons with Fluoro-Jade and acetylated alpha-tubulin (marker of microtubule stability) as described below. Three slices for each animal's brain in each experimental group/marker were matched for anteroposterior level and processed.

### Fluoro-Jade:

Labeling was carried out as previously described (Ravizza et al., 2008). Briefly, sections were dried in ethanol (100%, 75% and 50%) and rehydrated in distilled water. Then, they were incubated in 0.06% potassium permanganate, washed in distilled water and transferred to 0.001% Fluoro-Jade staining solution. Sections were then rinsed in distilled water, mounted onto gelatin-coated slides, dried, immersed in xylene and coverslipped.

### Acetylated alpha-tubulin:

Slices were blocked for 30 min at 4°C in blocking buffer (0.1% Triton-X100, 3% BSA in PBS; Invitrogen) then incubated with primary antibody against acetylated alpha-tubuline 1:2000 (#24610 Abcam) overnight at 4 C. After washing with PBS, slices were incubated with secondary antibody 1:500 (Alexa Fluor 488 anti-rabbit; Jackson Immunoresearch Laboratories, Inc., USA) for 1 h at room temperature and mounted on slides with fluorescein mounting medium (Vectashield, Vector laboratories, USA).

Furthermore, cell loss was quantified as previously described (Noe et al., 2013) by Nissl staining. Briefly, images of the septal pole of the hippocampus ipsilateral to the injected amygdala were captured at 20x magnification using a BX61 microscope equipped with motorized platform (Olympus, Germany) and images were digitized. Three slices at the same antero-posterior levels were matched for each control and experimental mouse. Neuronal cell loss was quantified by reckoning the number of Nissl-stained neurons in CA1 and CA3/CA4 pyramidal cell layers and the hilar interneurons. Data obtained in each of the 3 slices per mouse were averaged, thus providing a single value for each brain area/mouse, and this value was used for statistical analysis.

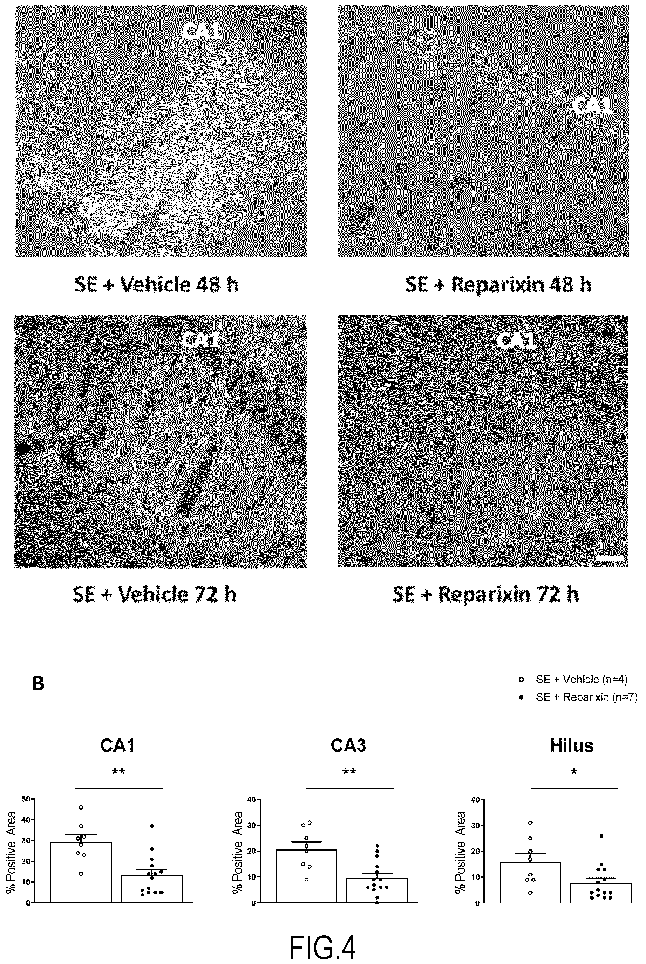

Figure 4A depicts representative sections stained for detection of acetylated alpha-tubulin in the CA1 pyramidal cell layer while signal quantification is reported in Figure 4B. Data show that administration of reparixin significantly reduced the immunohistochemical signal of acetylated alpha-tubulin, in the mouse hippocampus, as assessed 48 h and 72 h after SE. Vehicle-treated mice (n=4; one mouse died during SE and 1 mouse did not show a good quality signal); reparixin-treated mice (n=7) and sham mice (n=6): hippocampi in both hemispheres were used for quantification in each mouse.

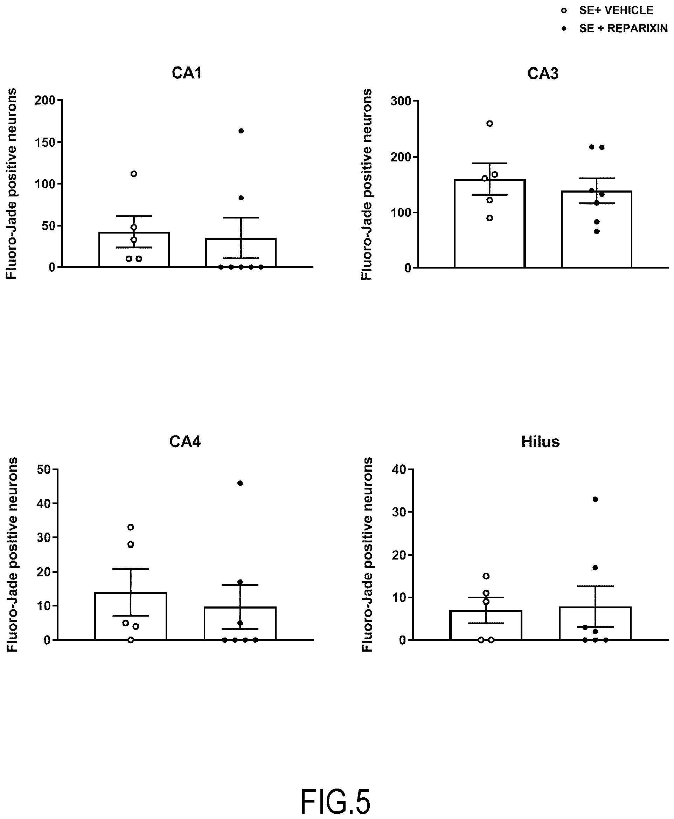

Figure 5 shows the number of Fluoro-Jade-positive degenerating neurons in CA1, CA3, CA4 pyramidal cell layers and hilus inmice exposed to SE and treated with reparixin or vehicle. Notably, in CA1 pyramidal cell layer 5 out of 7 mice treated with reparixin did not show Fluoro-Jade positive neurons while 5 out of 5 mice showed neurodegeneration in the vehicle group (p<0.05 by Fisher test). In CA4, 4 out of 7 mice treated with reparixin did not show Fluoro-Jade positive neurons while only 1 out of 5 mice did not show neurodegeneration in the vehicle group (p=0.29 by Fisher test).

These results demonstrate that the administration for reparixin protects neuronal cells from acute injury induced by SE.

### Example 4

### Effect of reparixin on established seizures in chronic epileptic mice

The objective of this experiment was to assess whether reparixin affects seizure frequency or duration in chronic epileptic mice with established spontaneous seizures. Status epilepticus was induced in 18 mice prepared as described in Example 1a) by amygdala injection of kainic acid and EEG was recorded for 14 days to determine disease onset as described in Example 2.

Then, mice were disconnected from EEG and returned to their home cages until day 45 at which time they were again EEG recorded for 14 days to determine their individual baseline of spontaneous seizures. At the end of this period, mice were temporarly disconnected for minipumps implantation. Minipumps (ALZET model 2002; 0.5µl/h) were filled with Reparixin (7.5/mg/h/kg, n=9) or its vehicle (n=9) according to the manifacturer's instructions and implanted subcutaneously in the back of the mice under general gas anaesthesia (1-3% isoflurane in O2). Upon awakening from anesthesia, mice were returned to their home cages for monitoring the EEG for additional 14 days during treatment. At the end of treatment period (as determined by minipump exhaustion) venous blood was collected from the cheek and plasma was isolated for drug level measurement. Mice were subsequently monitored during a drug wash-out period of 14 days.

A group of sham mice (not exposed to SE but handled as experimental mice) were implanted with minipumps filled with vehicle (n=6).

At the end of EEG recording, mice and their sham controls were killed and their brains were fixed in 10% formalin in phosphate buffered saline (PBS, pH 7.4) and embedded in paraffin for histological analysis.

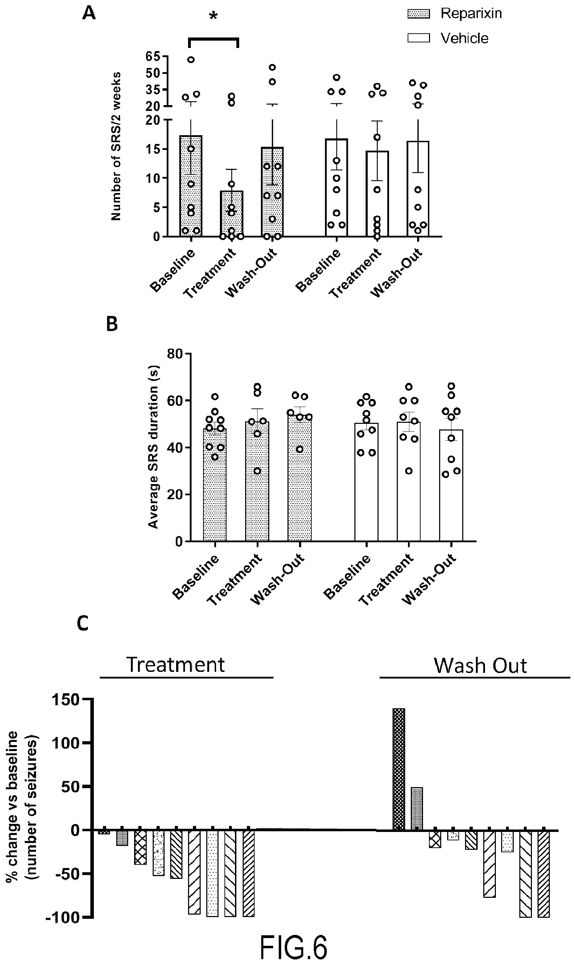

Figure 6 shows the number of seizures SRS (A) and their average duration (B) in chronic epileptic mice treated with reparixin or vehicle. Data show that reparixin reduces by about 50% the number of spontaneous seizures compared to pre-injection baseline (n= 9, p<0.05 by one-way ANOVA followed by Tukey's test). Seizures returned to pre-injection baseline level upon drug discontinuation. The average SRS duration was not modified.

Vehicle-treated epileptic mice (n=9) did not show any seizure reduction during or after vehicle treatment.

Figure 6C depicts the waterfall plot reporting reparixin effect in each epileptic mouse: 7 out of 9 mice were considered "responders" since they displayed at least 30% seizure reduction during treatment when compared to their pre-treatment baseline.

These results demonstrate that reparixin has an anticonvulsive effect also on chronic seizure in established epilepsy.

## Claims

1. A CXCL8 inhibitor, a pharmaceutically acceptable salt therof or a prodrug thereof, for use in the prevention or treatment of seizures or in the prevention of the onset or progression of epilepsy in an individual.

2. The CXCL8 inhibitor for use as claimed in claim 1, wherein said seizures are acute symptomatic seizures.

3. The CXCL8 inhibitor for use as claimed in claim 1, wherein said seizures are established epilepsy seizures.

4. The CXCL8 inhibitor for use as claimed in claim 1, wherein said seizures are status epilepticus seizures.

5. The CXCL8 inhibitor for use as claimed in claim 4, wherein said seizures de novo status epilepticus seizures.

6. The CXCL8 inhibitor for use as claimed in claimed 1 to 5, wherein said CXCL8 inhibitor is a CXCR1receptor inhibitor or a dual CXCR1 and CXCR2 receptor inhibitor.

7. The CXCL8 inhibitor for use as claimed in claims 1 to 6, wherein said CXCL8 inhibitor is a compound of formula (I): 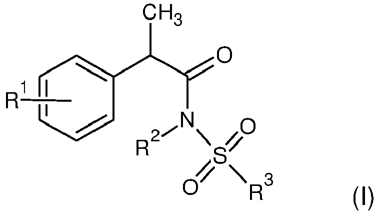 wherein
R¹ is selected from a linear or branched C₁-C₆ alkyl, benzoyl, phenoxy, and trifluoromethanesulfonyloxy;
R² is selected from hydrogen and linear or branched C₁-C₃ alkyl; andR³ is a linear or branched C₁-C₆ alkyl or trifluoromethyl,
a pharmaceutically acceptable salt therof or a prodrug thereof.

8. The CXCL8 inhibitor for use as claimed in claim 7, wherein said CXCL8 inhibitor is selected from:
- 2-(4-isobutylphenyl)propionyl methansulfonamide, preferably R-(-)-2-(4-isobutylphenyl)propionyl methansulfonamide and pharmaceutically acceptable salts thereof, more preferably the lysine salt thereof, and
- 2-(4-trifluoromethanesulfonyloxy)phenyl]-N-methanesulfonyl propionamide, preferably R(-)-2-(4-trifluoromethanesulfonyloxy)phenyl]-N-methanesulfonyl propionamide and pharmaceutically acceptable salts thereof, more preferably the sodium salt thereof.

9. The CXCL8 inhibitor for use as claimed in claims 1 to 6, wherein said CXCL8 inhibitor is a compound of formula (II): 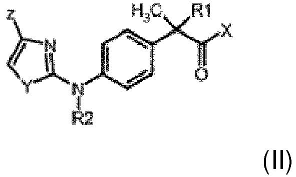 wherein:
R1 is hydrogen;
X is OH;
R2 is hydrogen or linear C1-C4 alkyl,
Y is a heteroatom selected from S, O and N,
Z is selected from linear or branched C1-C4 alkyl, linear or branched C1-C4 alkoxy, halo C1-C3 alkyl and halo C1-C3 alkoxy,
a pharmaceutically acceptable salt therof or a prodrug thereof.

10. The CXCL8 inhibitor for use as claimed in claim 9, wherein said CXCL8 inhibitor is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

11. The CXCL8 inhibitor for use as claimed in any one of claims 1 to 6, wherein said CXCL8 inhibitor is selected from the list consisting of
- the anti IL-8 antibodies ABCream, BMS-986253 and ABX-IL-8;
- RP-72, PAC-G-31-P, SCH-N, Navarixin, having formula 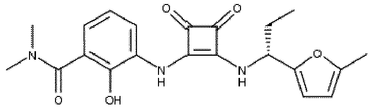 SX-517, having formula 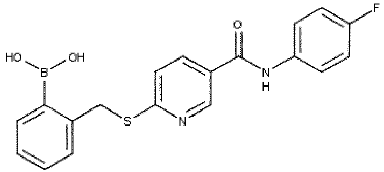 SX-576, having formula 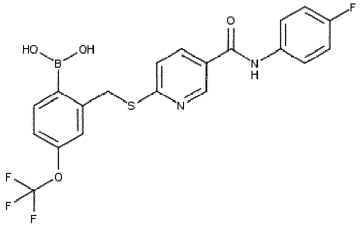 compounds having formula 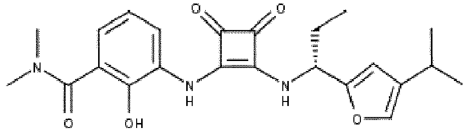 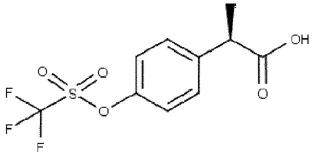 or 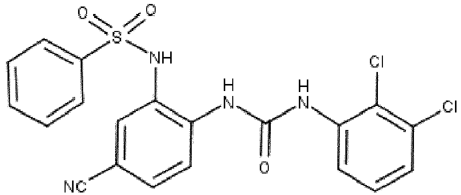 and
5-[3-(2-Fluorophenyl)ureido]-1-(2-hydroxypropyl)-1H-pyrazole-4-carboxylic acid ethyl ester
5-[3-(3-Fluorophenyl)ureido]-1-(2-hydroxypropyl)-1H-pyrazole-4-carboxylic acid ethyl ester
3-[2-[1(R)-(4-Bromofuran-2-yl)propylamino]-3,4-dioxo-1-cyclobutenylamino]-2-hydroxy-N,N-dimethylbenzamide
3-[2-[1(R)-(4-Chlorofuran-2-yl)propylamino]-3,4-dioxo-1-cyclobutenylamino]-2-hydroxy-N,N-dimethylbenzamide Trifluoromethanesulfonic acid 4-[1(R)-(N-isopropylcarbamoyl)ethyl]phenyl ester 2-Hydroxy-3-[4-[1(R)-(4-isopropylfuran-2-yl)propylamino]-1-oxo-1,2,5-thiadiazol-3-ylamino]-N,N-dimethylbenzamide
3-(2-Chlorophenylamino)-7-nitro-4H-1,2,4-benzothiadiazin-5-ol 1,1-dioxide 1-[3-[4-[3-(4-Fluorophenyl)isoxazol-5-yl]phenoxy]propyl]-4-methylpiperazine N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) as described in claims 7 to 10.

12. A pharmaceutical composition comprising a CXCL8 inhibitor for use as claimed in claims 1 to 11 and at least one inert pharmaceutically acceptable excipient, for use in the prevention and/or treatment of seizures or in the prevention of the onset or progression of epilepsy.

13. The CXCL8 inhibitor or the pharmaceutical composition for use as claimed in claims 1 to 12, wherein the CXCL8 inhibitor is administered in combination with at least another drug, wherein said at least another drug is administered simultaneously, sequentially or separately.

14. The CXCL8 inhibitor or the pharmaceutical composition for use as claimed in claim 13, wherein the at least another drug is selected from benzodiazepines and antiepileptic drugs, more preferably from diazepam, lorazepam, phenytoin and valproic acid (VPA).
